## Europäisches Patentamt
## European Patent Office
## Office européen des brevets

(11) Veröffentlichungsnummer: **0 073 881**
**B1**

(12) # EUROPÄISCHE PATENTSCHRIFT

(45) Veröffentlichungstag der Patentschrift:
**17.09.86**

(51) Int. Cl.⁴: **A 61 N 1/05**

(21) Anmeldenummer: **82104847.7**

(22) Anmeldetag: **03.06.82**

(54) **Kabelzuleitung für Herzschrittmacher-Elektroden.**

(30) Priorität: **03.09.81 DE 3134896**

(43) Veröffentlichungstag der Anmeldung:
**16.03.83 Patentblatt 83/11**

(45) Bekanntmachung des Hinweises auf die Patenterteilung:
**17.09.86 Patentblatt 86/38**

(84) Benannte Vertragsstaaten:
**CH DE FR GB LI NL**

(56) Entgegenhaltungen:
**EP - A - 0 054 781**
**EP - A - 0 064 289**
**DE - C - 2 134 926**
**FR - A - 1 571 849**
**FR - A - 2 133 717**
**FR - A - 2 463 625**
**US - A - 3 333 045**
**US - A - 3 590 822**
**US - A - 3 905 828**

(73) Patentinhaber: **W.C. Heraeus GmbH,**
**Heraeusstrasse 12 - 14, D-6450 Hanau / Main (DE)**

(72) Erfinder: **Aldinger, Fritz, Dr., Barbarossastrasse 44,**
**D-6458 Rodenbach 2 (DE)**
Erfinder: **Bischoff, Albrecht, Dr., Tannenweg 10,**
**D-6454 Bruchköbel (DE)**
Erfinder: **Keilberth, Richard, Löwensteinring 25,**
**D-8764 Kleinheubach (DE)**
Erfinder: **Sperner, Franz, Dr., Friedensstrasse 59,**
**D-6450 Hanau am Main (DE)**

(74) Vertreter: **Heinen, Gerhard, Dr., Heraeusstrasse 12-14,**
**D-6450 Hanau/Main (DE)**

ACTORUM AG

## Beschreibung

Die Erfindung bezieht sich auf eine Kabelzuleitung für eine Herzschrittmacher-Elektrode, mit einem Hüllrohr aus elektrisch isolierendem, elastischem Werkstoff und wenigstens einem darin angeordneten Leiter für elektrische Reizstrom-Impulse, wobei der Leiter ein durch Ziehen hergestellter Manteldraht ist, der einen Kern aus einem Metall hoher elektrischer Leitfähigkeit und einen Mantel aus einem korrosionsfesten, nicht-toxischen Metall aufweist.

Eine Kabelzuleitung für eine Herzschrittmacher-Elektrode ist aus der US-PS 3 749 101 bekannt. Als Werkstoff für das Hüllrohr wird Siliconkautschuk verwandt. Der in dem elastischen Hüllrohr angeordnete drahtförmige Leiter besteht aus einer korrosionsfesten Legierung aus 20 bis 50% Co, 15 bis 30% Cr, 5 bis 10% Ni, bis zu 18% Fe, 1 bis 10% Mo, bis zu 3% Mn, bis zu 0,3% C und 0,01 bis 0,09% Be.

Aus der US-PS 4 273 137 ist ferner eine Kabelzuleitung für eine Herzschrittmacher-Elektrode bekannt. In einem Hüllrohr aus Silicongummi ist ein elektrischer Leiter angeordnet, der aus einer Vielzahl von elektrisch leitfähigen Fasern besteht, wobei diese Fasern einen Mantel aus einer korrosionsfesten, nicht-toxischen Metall-Legierung, wie rostfreier Stahl oder eine Kobaltbasis-Legierung, aufweisen, der einen Kern aus einem Metall hoher elektrischer Leitfähigkeit umhüllt. Als Kernwerkstoffe werden Silber, Kupfer, Silberbasis-Legierungen oder Kupferbasis-Legierungen verwendet. Die Vielzahl der elektrisch leitfähigen Fasern ist in einer Hülle angeordnet, die wiederum aus dem gleichen korrosionsfesten, nicht-toxischen Metall besteht wie der Mantelwerkstoff der einzelnen Faser. Die einzelne Faser besteht aus einem als Mantel dienenden Röhrchen, das mit dem Metall hoher elektrischer Leitfähigkeit gefüllt ist.

Schliesslich ist aus der US-PS 3 333 045 eine Kabelzuleitung für eine Herzschrittmacher-Elektrode bekannt, bei der in einem Hüllrohr aus Silicongummi ein durch Ziehen hergestellter Manteldraht als Leiter angeordnet ist. Der Leiter weist einen Kern aus Silber und einen Mantel aus Platin auf

Aufgabe der Erfindung ist es, eine Kabelzuleitung zu schaffen, bei der elektrische Verlustleistungen auf ein Minimum reduziert sind, und die eine gute Biokompatibilität sicherstellt.

Gelöst wird diese Aufabe für eine Kabelzuleitung der eingangs charakterisierten Art erfindungsgemäss dadurch, dass der Mantel aus einem Metall aus der Gruppe Tantal, Titan, Zirkonium, Niob, Titan-Basis-Legierung, Platin-Iridium-Legierung, Platin-Palladium-Legierung und Platin-Rhodium-Legierung besteht, wobei die Mantelwandstärke im Bereich von 0,0025 bis 0,035 mm liegt und der Kerndurchmesser 0,04 bis 0,3 mm beträgt.

Es hat sich besonders bewährt, den Mantel aus Tantal, Titan, Zirkonium, Niob oder einer Titan-Basis-Legierung herzustellen, dessen Aussenoberfläche elektrisch isolierend ausgebildet ist, vorzugsweise anodisiert ist.

Als Werkstoffe für den Kern haben sich insbesondere Kupfer und Kupfer-Legierungen als geeignet erwiesen. Als wesentliche Legierungselemente neben Kupfer kommen insbesondere Zr, Ti, Be, Fe, P, Zn oder Sn in Frage. Beispiele für derartige Legierungen sind CuZr0,15, CuTi4, CuBe2, CuBe1,7, CuBe0,7, CuZn28, CuZn37, CuSn6, CuSn8 oder CuFe2.

Als Kabelzuleitung hat sich insbesondere eine solche bewährt, bei der der Kern des Manteldrahtes aus CuZr0,15 oder CuBe2 und der Mantel aus Tantal bestehen, dessen Aussenoberfläche anodisiert ist.

Als Mantelwerkstoffe haben sich Pt-Ir-Legierungen mit bis zu 40% Ir, Rest Pt, Pt-Pd-Legierungen mit bis zu 50% Pd, Rest Pt sowie Pt-Rh-Legierungen mit bis zu 40% Rh, Rest Pt bewährt, vorteilhafterweise Legierungen aus PtIr10, PtPd10 und PtRh10. Ein bevorzugtes Beispiel für Mantelwerkstoffe aus Titan-Basis-Legierungen sind die Legierungen TiA16V4 und TiA15Fe2,5.

Das elastische Hüllrohr, in dem der Manteldraht angeordnet ist, besteht aus Kunststoff, wie beispielsweise aus Siliconkautschuk oder Polyurethan. Es ist so elastisch und flexibel, dass seine Einführung in die Herzkammer allein durch die Mitnahme durch den Blutstrom möglich ist.

Erfindungsgemäss ausgebildete Kabelzuleitungen zeichnen sich, wie Ermüdungstests ergeben haben, durch hohe Standzeiten aus. Ausserdem ergibt sich eine sehr lange Lebensdauer der für die elektrischen Reizstrom-Impulse erforderlichen Batterien.

Die Biokompatibilität des drahtförmigen Leiters wird durch die Verwendung der in Anspruch 1 genannten Mantelwerkstoffe sichergestellt. Durch die Nichtleitendmachung der Aussenoberfläche der in Anspruch 2 genannten Mantelwerkstoffe, vorzugsweise durch Anodisieren, ist sichergestellt, dass bei Beschädigung oder durch andere Ursachen eintretendes Undichtwerden des Kunststoffhüllrohres Fehlstimulation des Herzens vermieden werden kann.

Eine Interdiffusion des Kernwerkstoffes an die äussere Oberfläche des Mantels konnte bei dem erfindungsgemäss durch Ziehen hergestellten Leiter nicht festgestellt werden.

In Fig. 1 ist ein Ausführungsbeispiel einer erfindungsgemässen Kabelzuleitung dargestellt.

Fig. 2 zeigt einen Querschnitt durch den Manteldraht.

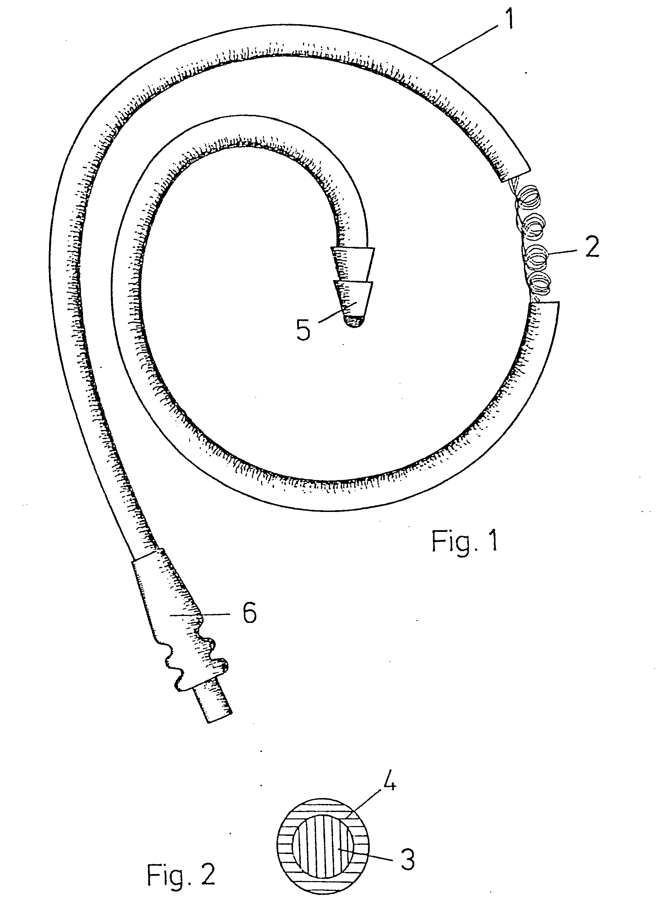

Mit der Bezugsziffer 1 ist in Fig. 1 das aus Kunststoff, z.B. Siliconkautschuk, bestehende Hüllrohr bezeichnet, in dem der Leiter 2 für elektrische Reizstrom-Impulse angeordnet ist. In diesem Ausführungsbeispiel ist der Leiter 2 aus vier gewendelten Manteldrähten gebildet, wobei die Stärke der einzelnen Manteldrähte 0,11 mm beträgt. Der Kern eines Manteldrahts besteht aus CuZr0,15, der Mantel aus Ta, dessen Aussenoberfläche anodisiert ist. Für eine derartige 60 cm lange Kabelzuleitung – diese Länge entspricht den üblicherweise zum Einsatz gelangenden Kabelzuleitungen – wurde ein elektrischer Widerstand von 1,75 Ω gemessen. Ein Wert, der um etwa eine Grössenordnung kleiner ist als auf dem Markt befindliche Kabelzuleitungen. Mit 5 ist die Herzschrittmacher-Elektrode bezeichnet, mit 6 die Anschlusssteckvorrichtung, die an das Herzschrittmacher-Steuergerät angeschlossen wird.

Bei dem in Fig. 2 dargestellten Manteldraht-Querschnitt ist mit 3 der Kern bezeichnet und mit 4 der Mantel. Die Wandstärke des Mantels beträgt im Ausführungsbeispiel 0,012 mm, der Durchmesser des Kerns 0,086 mm. Übliche Wandstärken für den Mantel liegen im Bereich von 0,0025 bis 0,035 mm, Kerndurchmesser liegen im Bereich von 0,04 bis 0,3 mm.

## Patentansprüche

1. Kabelzuleitung für eine Herzschrittmacher-Elektrode (5) mit einem Hüllrohr (1) aus elektrisch isolierendem, elastischem Werkstoff und wenigstens einem darin angeordneten Leiter (2) für elektrische Reizstrom-Impulse, wobei der Leiter ein durch Ziehen hergestellter Manteldraht ist, der einen Kern (3) aus einem Metall hoher elektrischer Leitfähigkeit und einen Mantel (4) aus einem korrosionsfesten, nicht-toxischen Metall aufweist, dadurch gekennzeichnet, dass der Mantel aus einem Metall aus der Gruppe Tantal, Titan, Zirkonium, Niob, Titan-Basis-Legierung, Platin-Iridium-Legierung, Platin-Palladium-Legierung und Platin-Rhodium-Legierung besteht, wobei die Mantelwandstärke im Bereich von 0,0025 bis 0,035 mm liegt und der Kerndurchmesser 0,04 bis 0,3 mm beträgt.

2. Kabelzuleitung nach Anspruch 1, dadurch gekennzeichnet, dass die Aussenoberfläche des Mantels aus Tantal, Titan, Zirkonium, Niob oder einer Titan-Basis-Legierung elektrisch isolierend ausgebildet ist.

3. Kabelzuleitung nach den Ansprüchen 1 oder 2, dadurch gekennzeichnet, dass der Kern aus Kupfer oder einer Kupferlegierung besteht.

4. Kabelzuleitung nach Anspruch 3, dadurch gekennzeichnet, dass der Kernwerkstoff neben Kupfer als weiteres Legierungselement Zr, Ti, Be, Fe, P, Zn oder Sn enthält.

5. Kabelzuleitung nach den Ansprüchen 3 und 4, dadurch gekennzeichnet, dass der Kern aus CuZr0,15, CuTi4, CuBe2, CuBe1,7, CuBe0,7, CuZn28, CuZn37, CuSn6, CuSn8 oder CuFe2 besteht.

6. Kabelzuleitung nach den Ansprüchen 1 bis 5, dadurch gekennzeichnet, dass der Kern aus CuZr0,15 oder CuBe2 und der Mantel aus Tantal bestehen, dessen Aussenoberfläche anodisiert ist.

7. Kabelzuleitung nach einem oder mehreren der vorhergehenden Ansprüche, dadurch gekennzeichnet, dass der Mantel aus einer Pt-Ir-Legierung mit bis zu 40% Ir oder einer Pt-Rh-Legierung mit bis zu 40% Rh, oder einer Pt-Pd-Legierung mit bis zu 50% Pd, Rest Platin besteht.

8. Kabelzuleitung nach Anspruch 7, dadurch gekennzeichnet, dass der Mantel aus der Legierung PtIr10, PtId10 oder PtRh10 besteht.

## Claims

1. Supply lead for a heart pacemaker electrode (5) comprising a sheath tube (1) of electrically insulating elastic material and at least one conductor (2) situated therein and intended for electrical excitation current pulses, wherein the conductor is a sheathed wire produced by drawing which has a core (3) of a metal of high electrical conductivity and a sheath (4) of an anticorrosive non-toxic metal, characterised in that the sheath is formed from a metal of the group tantalum, titanium, zirconium, niobium, titanium-based alloy, platinum-iridium alloy, platinum-palldium alloy and platinum-rhodium alloy, the sheath gauge thickness lying in the range from 0.0025 to 0.035 mm and the core diameter amounting to 0.04 to 0.3 mm.

2. Supply lead according to claim 1, characterised in that the outer surface of the sheath of tantalum, titanium, zirconium, niobium or titanium based alloy, is made electrically insulating.

3. Supply lead according to claims 1 or 2, characterised in that the core is formed from copper or a copper alloy.

4. Supply lead according to claim 3, characterised in that apart from copper, the core material contains Zr, Ti, Be, Fe, P, Zn or Sn as another alloying element.

5. Supply lead according to claims 3 and 4, characterised in that the core is formed from CuZr 0.15, CuTi 4, CuBe 2, CuBe 1.7, CuBe 0.7, CuZn 28, CuZn 37, CuSn 6, CuSn 8 or CuFe 2.

6. Supply lead according to claims 1 to 5, characterised in that the core consists of CuZr 0.15 or CuBe 2, and the sheath of tantalum whose external surface is anodised.

7. Supply lead according to one of more of the preceding claims, characterised in that the sheath comprises a Pt-Ir alloy containing up to 40% of Ir or a Pt-Rh alloy containing up to 40% of Rh, or a Pt-Pd alloy containing up to 50% of Pd, the remainder being platinum.

8. Supply lead according to claim 7, characterised in that the sheath comprises the alloy PtIr 10, PtPd 10 or PtRh 10.

## Revendications

1. Câble de connexion pour une électrode de stimulateur cardiaque (5), comportant un tube enveloppe (1) en matériau élastique électriquement isolant et au moins un conducteur (2) disposé dans ce tube et servant à transmettre des impulsions électriques de stimulation, le conducteur étant un fil gainé produit par tréfilage qui possède un noyau (3) en métal de haute conductivité électrique et une gaine (4) en métal non toxique résistant à la corrosion, caractérisé en ce que la gaine est faite d'un métal du groupe comprenant le tantale, le titane, le zirconium, le niobium, les alliages à base de titane, les alliages platine-iridium, les alliages platine-palladium et les alliages platine-rhodium, l'épaisseur de paroi de la gaine étant de l'ordre de 0,0025 à 0,035 mm et le diamètre du noyau étant de 0,04 à 0,3 mm.

2. Câble selon la revendication 1, caractérisé en ce que la surface extérieure de la gaine en tantale, titane, zirconium, niobium ou alliage à base de titane, est électriquement isolante.

3. Câble selon la revendication 1 ou 2, caractérisé en ce que le noyau est en cuivre ou alliage de cuivre.

4. Câble selon la revendication 3, caractérisé en ce que le matériau du noyau contient, comme autre composant d'alliage, en plus du cuivre, Zr, Ti, Be, Fe, P, Zn ou Sn.

5. Câble selon les revendications 3 et 4, caractérisé en ce que le noyau est en CuZr0,15, CuTi4, CuBe2, CuBe1,7, CuBe0,7, CuZn28, CuZn37, CuSn6, CuSn8 ou CuFe2.

6. Câble selon les revendications 1 à 5, caractérisé en ce que le noyau est en CuZr0,15 ou CuBe2 et la gaine est en tantale et présente une surface extérieure anodisée.

7. Câble selon une ou plusieurs des revendications précédentes, caractérisé en ce que la gaine est faite d'un alliage Pt-Ir contenant jusqu'à 40% Ir, d'un alliage Pt-Rh contenant jusqu'à 40% Rh ou d'un alliage Pt-Pd contenant jusqu'à 50% Pd, reste platine.

8. Câble selon la revendication 7, caractérisé en ce que la gaine est faite de l'alliage PtIr10, PtPd10 ou PtRh10.

Fig. 1

Fig. 2